(19) Europäisches Patentamt
European Patent Office
Office européen des brevets

(11) Veröffentlichungsnummer: **0 520 325 A1**

(12) **EUROPÄISCHE PATENTANMELDUNG**

(21) Anmeldenummer: **92110332.1**

(51) Int. Cl.5: **A61K 31/165**, A61K 31/44

(22) Anmeldetag: **19.06.92**

(30) Priorität: **28.06.91 CH 1917/91**

(43) Veröffentlichungstag der Anmeldung:
**30.12.92 Patentblatt 92/53**

(84) Benannte Vertragsstaaten:
**AT BE CH DE DK ES FR GB GR IT LI LU MC NL PT SE**

(71) Anmelder: **F.HOFFMANN-LA ROCHE & CO. AKTIENGESELLSCHAFT**
**Postfach 3255**
**CH-4002 Basel(CH)**

(72) Erfinder: **Haefely, Willy**
**125 Unterer Rebbergweg**
**CH-4153 Reinach(CH)**

(74) Vertreter: **Grossner, Lutz, Dr. et al**
**Grenzacher Strasse 124 Postfach 3255**
**CH-4002 Basel(CH)**

(54) **Verwendung von Monoaminoxidase-B-Hemmern zur Verhütung und Behandlung von Entzugserscheinungen nach Alkohol- und Drogenmissbrauch.**

(57) Gewisse MAO-B-Hemmer können zur Behandlung und Verhütung von Entzugserscheinungen, wie sie nach Rauschmittel-(z.B. Alkohol-) Genuss auftreten, verwendet werden.

EP 0 520 325 A1

Monoaminoxidase (MAO)-Hemmer werden zur Behandlung der Parkinson'schen Erkrankung und von Depressionen eingesetzt. Gewisse MAO-Hemmer hemmen überwiegend oder selektiv die Monoaminoxidase A oder die Monoaminoxidase B.

Verbindungen, die überwiegend oder selektiv die Monoaminoxidase B hemmen (hier als Monoaminoxidase B-(MAO-B)-Hemmer bezeichnet), werden zur Behandlung der Parkinson-Symptomatik eingesetzt. Es wurde nun gefunden, dass solche Verbindungen auch zur Behandlung und Verhütung von Entzugserscheinungen, die nach wiederholtem Konsum von Suchtmitteln auftreten bzw. zur Herstellung von Heilmitteln für die Behandlung oder Verhütung von Entzugserscheinungen, die nach dem Absetzen von Suchtmitteln auftreten, verwendet werden können.

Beispiele solcher Suchtmittel sind insbesondere Alkohol (Aethylalkohol), sowie Tabakerzeugnisse und Drogen wie Cocain und Amphetamine. Es wurde gefunden, dass die Verabreichung von MAO-B-Hemmern an Personen, die solche Mittel konsumieren, das für Suchtzustände charakteristische Verlangen nach weiterem Konsum vermindert.

MAO-B-Hemmer sind bekannt und beispielsweise in den GB-Patentschriften 2 135 998 und 2 163 746 beschrieben. Als erfindungsgemäss verwendbare MAO-B-Hemmer kommen insbesondere Verbindungen der Formel

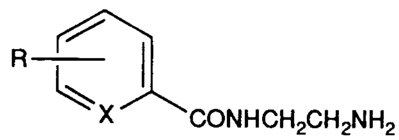

I

worin X N oder CH und R Halogen, insbesondere Chlor, ist und Säureaditionssalze davon in Betracht.

Eine bevorzugte Verbindung für die erfindungsgemässe Verwendung ist das N-(2-Aminoäthyl)-5-chlorpyridin-2-carboxamid und dessen Salze wie das Hydrochlorid.

Weitere Beispiele solcher Verbindungen sind:

N-(2-Aminoäthyl)-5-brompyridin-2-carboxamid,

N-(2-Aminoäthyl-6-chlorpyridin-2-carboxamid,

2-Aminoäthyl-p-chlorbenzamid,

2-Aminoäthyl-p-brombenzamid,

2-Aminoäthyl-p-fluorbenzamid und Säureadditionssalze davon.

Beispiele solcher Säureadditionssalze sind Salze mit anorganischen und organischen Säuren, wie Salzsäure, Bromwasserstoffsäure, Salpetersäure, Schwefelsäure, Phosphorsäure, Citronensäure, Ameisensäure, Maleinsäure, Essigsäure, Bernsteinsäure, Weinsäure, Methansulfonsäure, p-Toluolsulfonsäure und dergleichen.

Die vorliegende Erfindung betrifft demnach die Verwendung von Verbindungen der Formel I und Säureadditionssalzen davon zur Herstellung von Heilmitteln zur Behandlung und Verhütung von Entzugserscheinungen, die nach dem Konsum von Rauschmitteln, wie Alkohol, Tabakerzeugnissen und Cocain auftreten. Diese Heilmittel können als pharmazeutische Präparate für die orale Verabreichung, z.B. in Form von Tabletten, Lacktabletten, Dragées, Hart- und Weichgelatinekapseln, Lösungen, Emulsionen oder Suspensionen, vorliegen, oder für rektale Verabreichung, z.B. in Form von Suppositorien, oder für parenterale Verabreichung, z.B. in Form von Injektionslösungen, vorliegen.

Zur Herstellung von Tabletten, Lacktabletten, Dragées und Hartgelatinekapseln können die Wirkstoffe mit pharmazeutisch inerten, anorganischen oder organischen Excipientien verabreicht werden. Als solche Excipientien kann mit z.B. für Tabletten, Dragées und Hartgelatinekapseln, Lactose, Maisstärke oder Derivate davon, Talk, Stearinsäure oder deren Salze etc. verwenden.

Für Weichgelatinekapseln eignen sich als Excipientien z.B. vegetabile Oele, Wachse, Fette, halbfeste und flüssige Polyole etc.

Zur Herstellung von Lösungen und Sirupen eignen sich als Excipientien z.B. Wasser, Polyole, Saccharose, Invertzucker, Glukose etc.

Für Injektionslösungen eignen sich als Excipientien z.B. Wasser, Alkohole, Polyole, Glycerin, vegetabile Oele etc.

Für Suppositorien eignen sich als Excipientien z.B. natürliche oder gehärtete Oele, Wachse, Fette, halbflüssige oder flüssige Polyole etc.

Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

Erfindungsgemäss kann die Dosierung oder Wirkstoffe innerhalb weiter Grenzen variieren und ist den individuellen Gegebenheiten anzupassen. Im allgemeinen kommt bei oraler Verabreichung eine Tagesdosis von etwa 10 bis 100 mg des Wirkstoffs, z.B. einer Verbindung der allgemeinen Formel I in Betracht.

Die nachstehenden Beispiele erläutern die Erfindung weiter.

Beispiel 1

Gelatinesteck-Kapseln à 10 mg

Zusammensetzung:

| | | | |
|---|---|---|---|
| 1. | N-(2-Aminoäthyl)-5-chlorpyridin-2-carboxamid-hydrochlorid | | 11,82 mg*) |
| 2. | Milchzucker pulv. | | 74,18 mg |
| 3. | Maisstärke | | 40,00 mg |
| 4. | Talk | | 3,60 mg |
| 5. | Magnesiumstearat | | 0,40 mg |
| 6. | Milchzucker krist. | | 110,00 mg |
| | | Kapselfüllgewicht | 240,00 mg |

*) entspricht 10 mg Base

Herstellungsverfahren:

Die Bestandteile 1-5 werden gemischt und durch ein Sieb mit 0,5 mm Maschenweite gesiebt. Danach wird Bestandteil 6 zugefügt und gemischt. Die Mischung wird in Gelatinesteckkapseln geeigneter Grösse (z.B. Nr. 2) mit einem Einzelfüllgewicht von 240 mg abgefüllt.

Beispiel 2

Tablette à 10 mg

Zusammensetzung;

| | | | |
|---|---|---|---|
| 1. | N-(2-Aminoäthyl)-5-chlorpyridin-2-carboxamid-hydrochlorid | | 11,82 mg*) |
| 2. | Milchzucker pulv. | | 103,18 mg |
| 3. | Maisstärke | | 40,00 mg |
| 4. | Polyvinylpyrrolidon K 30 | | 15,00 mg |
| 5. | Maisstärke | | 25,00 mg |
| 6. | Talk | | 4,50 mg |
| 7. | Magnesiumstearat | | 0.50 mg |
| | | Tablettengewicht | 200,00 mg |

*) entspricht 10 mg Base

Verfahren:

Die Bestandteile 1-3 werden gemischt und durch ein Sieb mit 0,5 mm Maschenweite gesiebt. Diese

Pulvermis chung wird mit einer alkoholischen Lösung des Bestandteils 4 befeuchtet und geknetet. Die feuchte Masse wird granuliert, getrocknet und auf eine geeignete Korngrösse zugerüstet. Dem getrockneten Granulat werden nacheinander die Bestandteile 5, 6 und 7 zugefügt und gemischt. Die Mischung wird zu Tabletten geeigneter Grösse mit einem Sollgewicht von 200 mg verpresst.

**Patentansprüche**

**1.** Verwendung von Verbindungen der Formel

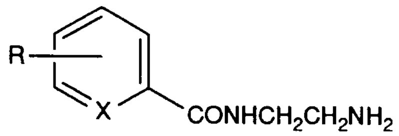

I

wobei X N oder CH und R Halogen darstellt,
oder Säureadditionssalzen davon zur Herstellung von Heilmitteln zur Behandlung und Verhütung von Entzugserscheinungen, die nach dem Konsum von Rauschmitteln auftreten.

**2.** Verwendung nach Anspruch 1 wobei das Rauschmittel Aethylalkohol ist.

**3.** Verwendung von N-(2-Aminoäthyl)-5-chlorpyridin-2-carboxamid oder einem Säureadditionssalz davon nach den Ansprüchen 1 oder 2.

Europäisches Patentamt

# EUROPÄISCHER RECHERCHENBERICHT

Nummer der Anmeldung

EP 92 11 0332

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | ALCOHOL AND ALCOHOLISM, Band 22, Nr. 1, 1987, Seiten 91-92, Pergamon Journals Ltd, GB; A. MORINAN: "Selective inhibition of monoamine oxidase type B by chlormethiazole" * Seite 91 * --- | 1-3 | A 61 K 31/165<br>A 61 K 31/44 |
| Y | THE PSYCHIATRIC CLINICS OF NORTH AMERICA, Band 7, Nr. 4, Dezember 1984, Seiten 713-728; R.B. RESNICK et al.: "Cocaine abuse and its treatment" * Seiten 723-724,727 * --- | 1-3 | |
| Y | THE JOURNAL OF CLINICAL PSYCHIATRY, Band 50, Nr. 11, Supplement, November 1989, Seiten 26-29; M.I. LINNOILA: "Anxiety and alcoholism" * Zusammenfassung; Seiten 28-29 * --- | 1-3 | |
| Y | ZHURNAL NEVROPATOLOGII I PSIKHIATRII, Band 91, Nr. 2, 1991, Seiten 79-83; A.E. BOBROV et al.: "Effectiveness of combined use of monoamine oxidase inhibitors and psycho therapy in the treatment of chronic alcoholism" * Zusammenfassung * --- | 1-3 | **RECHERCHIERTE SACHGEBIETE (Int. Cl.5)**<br>A 61 K |
| Y | "Index Nominum International Drug Directory", ed. Swiss Pharmaceutical Society, 1990/1991, Seite 780, Medpharm Scientific Publishers, Stuttgart, DE * Seite 780: "Nialamide" * --- | 1-3 | |
| Y | EP-A-0 385 210 (F. HOFFMANN-LA ROCHE AG) * Zusammenfassung; Seite 2 * --- -/- | 1-3 | |

Der vorliegende Recherchenbericht wurde für alle Patentansprüche erstellt

| Recherchenort | Abschlußdatum der Recherche | Prüfer |
|---|---|---|
| DEN HAAG | 23-08-1992 | KRAUTBAUER B. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)

Europäisches Patentamt

# EUROPÄISCHER RECHERCHENBERICHT

Nummer der Anmeldung

EP 92 11 0332

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | NEUROSCIENCE LETTERS, Band 127, Nr. 2, 24. Juni 1991, Seiten 247-250, Elsevier Scientific Publishers Ireland Ltd; G.S. SHAHI et al.: "1-Methyl-4-phenyl-1,2,3,6-tetrahydropyridine-induced neurotoxicity: partial protection against striato-nigral dopamine depletion in C57BL/6J mice by cigarette smoke exposure and by beta-naphthoflavone-pretreatment" * Seiten 248-249 * --- | 1-3 | |
| Y | PSYCHIATRY RESEARCH, Band 5, Nr. 3, 1981, Seiten 335-340, Elsevier/North-Holland Biomedical Press; H. PETURSSON et al.: "Urinary MAO inhibitor in psychiatric illness" * Seiten 335,338-339 * --- | 1-3 | |
| A | EP-A-0 099 775 (SOCIETE D'ETUDES SCIENTIFIQUES ET INDUSTRIELLES DE L'ILE DE FRANCE) * Seiten 1,7; Patentanspruch 2 * ----- | 1-3 | |
| | | | RECHERCHIERTE SACHGEBIETE (Int. Cl.5) |

Der vorliegende Recherchenbericht wurde für alle Patentansprüche erstellt

| Recherchenort | Abschlußdatum der Recherche | Prüfer |
|---|---|---|
| DEN HAAG | 23-08-1992 | KRAUTBAUER B. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument